# FASCICULE DE BREVET EUROPEEN

(11) **EP 1 235 797 B1**
(45) Date de publication et mention de la délivrance du brevet: **09.06.2004**
(21) Numéro de dépôt: 00976121.4
(22) Date de dépôt: 08.11.2000
(51) Int. Cl.: C07C 311/18, C07C 311/29, C07C 211/30, A61K 31/135, A61K 31/18

(54) **NOUVELLES ARALKYLE-1,2-DIAMINES POSSEDANT UNE ACTIVITE CALCIMIMETIQUE ET LEUR MODE DE PREPARATION**
ARALKYL-1,2-DIAMINEN MIT CALCIMIMETISCHER WIRKUNG UND VERFAHREN ZU IHRER HERSTELLUNG
ARALKYL-1,2-DIAMINES HAVING CALCIMIMETIC ACTIVITY AND PREPARATION MODE

(30) Priorité: 09.11.1999 FR 9914050
(43) Date de publication de la demande: 04.09.2002
(73) Titulaire: CENTRE NATIONAL DE LA RECHERCHE SCIENTIFIQUE (CNRS), 75794 Paris Cedex 16 (FR)
(72) Inventeur: RUAT, Martial, F-92340 Bourg-la-Reine (FR); POTIER, Pierre, Jean-Paul, Serge, Jacques, F-75007 Paris (FR); DODD, Robert, F-75009 Paris (FR); DAUBAN, Philippe, Marcel, F-91700 Sainte-Geneviève des Bois (FR); FAURE, Hélène, Véronique, F-91190 Gif-sur-Yvette (FR)
(74) Mandataire: Warcoin, Jacques
(86) Numéro de dépôt international: PCT/FR2000/003104
(87) Numéro de publication internationale: WO 2001/034562

(56) Documents cités:
- WO-A-97/41090
- P. E. MALIGRES ET AL: TETRAHEDRON LETT., vol. 38, no. 30, 1997, pages 5253-5256, XP004083291

## Description

La présente invention décrit une nouvelle classe de composés, les aralkyl-1,2-diamines, leur préparation, les compositions pharmaceutiques les comprenant et leur utilisation comme modulateur de l'activité des récepteurs aux ions (Ca²⁺)ₑ et (Mg²⁺)ₑ ou CaSR pour Calcium Sensing Receptor et comme médicament destiné de préférence au traitement des maladies ou des désordres physiologiques faisant intervenir la modulation de l'activité des CaSR.
Ces composés présentent donc une activité calcimimétique, c'est à dire capable de produire ou d'induire des réponses biologiques observées par les variations de la concentration des ions calcium extracellulaires (Ca²⁺)ₑ et des ions magnésium extracellulaires (Mg²⁺)ₑ.

Les ions (Ca²⁺)ₑ et (Mg²⁺)ₑ jouent un rôle majeur dans l'organisme car ils régulent l'homéostasie caicique dont dépendent les fonctions vitales de l'organisme. Ainsi, les hypercalcémies, c'est à dire des états ou les ions (Ca²⁺)ₑ sont au-dessus du seuil moyen, ont une incidence majeure sur de nombreuses fonctions telles que les fonctions cardiaques, rénales ou intestinales. Elles affectent profondément le système nerveux central (voir revue Chattopadhyay et al, Endocr. Review, 1998).

Les CaSR sont des protéines sensibles aux ions (Ca²⁺)ₑ et (Mg²⁺)ₑ et sont présentes dans les glandes parathyroïdiennes, thyroïdiennes, le rein, l'intestin, les poumons, les cellules osseuses, le cerveau, la moelle épinière, l'hypophyse, l'estomac, les kératinocytes (Brown et al, Nature, 1993 ; Ruat et al, Proc. Natl. Acad. Sci., USA, 1995 ; voir revue Brown et al, Ann. Rev. Med., 1998). Ces protéines sont codées par un seul gène isolé dans différentes espèces animales. Elles appartiennent à la famille des récepteurs couplés aux protéines G à sept domaines transmembranaires, et présentent des homologies de structure avec les récepteurs métabotropiques du glutamate, des récepteurs GABA_{B}, des récepteurs hypothétiques aux phéromones et du goût. Des mutations activatrices ou inhibitrices du gène chez l'homme sont responsables de maladies génétiques extrêmement graves qui provoquent des hypocalcémies ou des hypercalcémies (Pollack et al, Cell, 1993 ; Pollack et al, Nature Genetic, 1994 ; voir revue Brown et al, Ann. Rev. Med., 1998). Les fonctions liées à l'expression de ces protéines dans les tissus ne sont pas encore toutes connues et font l'objet d'une très grande activité de recherche, particulièrement en ce qui concerne les CaSR présents dans les glandes parathyroïdiennes, thyroïdiennes, le rein, l'intestin, la moelle épinière, le cerveau et les cellules osseuses.

Dans la glande parathyroïdienne, les CaSR modulent la sécrétion de l'hormone parathyroïdienne (PTH) qui est le principal régulateur de l'homéostasie calcique: l'augmentation des ions (Ca²⁺)ₑ dans le sérum va activer les CaSR présents sur les cellules de la glande parathyroïdienne et diminuer la sécrétion de l'hormone PTH.

L'ADN complémentaire codant pour le CaSR de rat a été isolé à partir d'une banque ADNc de striatum de rat (Ruat et al, Proc. Natl. Acad. Sci., 1995). Ce récepteur est identique au niveau de sa séquence en acides aminés à celui exprimé dans les autres tissus. Des cellules ovariennes de hamster chinois (CHO) transfectées exprimant le CaSR de rat (CHO(CaSR)) ont été caractérisées et les signaux chimiques (seconds messagers) induits par l'activation de ce récepteur ont été analysés. Ainsi, un test biochimique permettant de mesurer l'accumulation d'inositols phosphates tritiés [³H]IP en réponse à l'activation du récepteur a été mis au point (Ruat et al, J. Biol. Chem., 1996 ; Ferry et al, Biochem. Biophys. Res. Commun., 1997).

Il a été montré que les ions Ca²⁺, Mg²⁺, mais aussi Ba²⁺ dans des gammes de concentrations millimolaires stimulent les CaSR. L'activation des CaSR pourrait être induite dans le cerveau par les peptides β-amyloides, qui sont impliqués dans des maladies neurodégénératives telles que la maladie d'Alzheimer (Ye et al, J. Neurosci. Res., 1997).

Le composé NPS R-568 (1), ligand allostérique du CaSR, appartient à la première et, jusqu'à présent, seule famille de molécules organiques de petite taille (M < 600), interagissant avec ce récepteur. Cette arylalkylamine a été développée à partir de la structure de la Fendiline (2), un puissant activateur du CaSR de la glande parathyroïdienne.

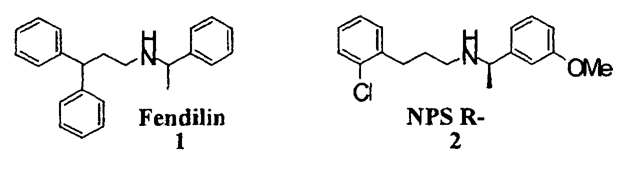

Les composés PHD selon la présente invention, ont une structure différente de ceux de cette famille par la présence d'un groupement 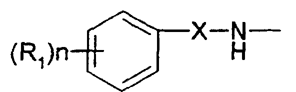 Ce groupement représente un site d'interaction de cette molécule avec les récepteurs CaSR.

L'hyperparathyroïdie secondaire est observée lors d'insuffisance rénale chronique et se caractérise par une hyperplasie des glandes parathyroïdiennes et une augmentation de la PTH circulante. L'insuffisance rénale est aussi accompagnée d'ostéodystrophie rénale qui se caractérise par des désordres osseux avec un fort ou un faible renouvellement de la masse osseuse (ostéitis fibrosa, osteomalacia). L'agent NPS-R-568 réduit ou élimine l'ostéitis fibrosa chez le rat (Wada et al, Kidney International, 1998) et réduit les concentrations de PTH chez des patients (hommes) souffrant d'insuffisance rénale chronique (Antansen et al, Kidney International, 1998). Ce composé a été utilisé par voie orale avec succès pour abaisser les concentrations de PTH et des ions Ca²⁺ libres sériques chez la femme ménopausée souffrant d'hyperparathyroïdie primaire (Silverberg et al, New Engl. J. Med., 1997). Dans une autre étude, le composé NPS-R-568 a permis de réduire entre 20-50 % la prolifération cellulaire observée dans la glande parathyroïdienne chez un modèle de rat reproduisant l'insuffisance rénale chronique (Wada et al, J. Clin. Invest., 1997). Ces études démontrent qu'un composé calcimimétique, actif vis-à-vis du récepteur au calcium présent sur la glande parathyroïde, peut-être considéré comme un outil thérapeutique intéressant pour traiter certaines formes d'hyperparathyroïdies primaires et secondaires.

Durant des essais cliniques, (Phase I - II) la société NPS Pharmaceutical a observé une bio-disponibilité faible du composé NPS-R-568 ainsi que des effets cliniques variables suivant les individus qui pourraient provenir de polymorphisme du gène codant pour le CaSR chez l'homme (Nemeth et al, Trends Endoc. Metab, 1999). Dans cette invention, les molécules synthétisées présentent des avantages par rapport au composé NPS-R-568 car leur structure fait apparaître plusieurs sites d'interaction avec le CaSR.

De plus, lors d'essais expérimentaux chez le rat, le composé NPS R-467, un composé de structure voisine du NPS R-568, s'est avéré plus sélectif vis à vis des récepteurs de la parathyroïde comparativement à ceux de la glande thyroïde. Cette sélectivité peut s'expliquer par des différences liées aux tissus ce qui suggère que des molécules calcimimétiques spécifiques d'un tissu peuvent être synthétisées et avoir des importances cliniques considérables. Du fait de la présence de nouveaux groupements capables d'interagir avec le CaSR ou avec son système de transduction, les molécules de la présente invention se révélent intéressantes en clinique.

L'ostéoporose est une maladie multifactorielle qui dépend notamment de l'âge et du sexe. Si les femmes ménopausées sont très fortement touchées, l'ostéoporose s'avère de plus en plus un problème chez l'homme âgé, et il n'existe pas pour l'instant de traitements vraiment satisfaisants. Son coût social pourrait s'alourdir encore dans les prochaines années, particulièrement dans notre société européenne ou la durée de vie s'allonge. L'ostéoporose est actuellement traitée par les oestrogènes, la calcitonine ou les biphosphonates qui préviennent la résorption osseuse sans stimuler une nouvelle croissance osseuse. Des données plus récentes démontrent que des augmentations intermittentes de la PTH ou de ses dérivés, sont efficaces dans le traitement de l'ostéoporose et permettent de remodeler l'os en stimulant la formation osseuse (Whitfield et al, 1999). Cette nouvelle voie thérapeutique du traitement de l'ostéoporose apparaît très intéressante bien que des problèmes majeurs soient liés à l'utilisation de l'hormone PTH tels que la voie d'injection, mais aussi l'apparition de tumeurs observées récemment durant des essais cliniques chez l'homme. La sécrétion intermittente de PTH endogène peut être obtenue par le blocage du récepteur au calcium. Le blocage de la sécrétion de PTH par les agonistes du CaSR peut être suivie par une augmentation rapide de la PTH (effet rebond), qui est alors bénéfique dans le traitement de l'ostéoporose.

Ainsi, les molécules décrites dans l'invention s'avèrent utiles pour moduler l'activité du CaSR dans la glande parathyroïde, la thyroïde, les cellules osseuses, l'estomac, le poumon, le rein, l'hypophyse, le cerveau. Elles s'avèrent aussi utilisables pour modifier l'activité de CaSR présents dans l'hypothalamus, les aires olfactives, l'hippocampe, pour traiter des maladies démyélinisantes associées à l'expression des CaSR dans les oligodendrocytes.

La présente invention décrit donc de nouvelles molécules destinées à traiter les désordres biologiques liés à des perturbations de l'activité des CaSR telles que les hyperparathyroïdies primaires et secondaires, l'ostéoporose, les maladies cardio-vasculaires, gastro-intestinales, endocrines, neurodégénératives, ou encore certains cancers où les ions (Ca²⁺)ₑ sont anormalement élevés. L'absence totale de molécules calcimimétiques en clinique, et les problèmes rencontrés en phase I-II pour les calcimimétiques de première génération, soulignent l'intérêt des molécules décrites dans l'invention, celles-ci ayant de plus un site d'interaction supplémentaire avec les récepteurs CaSR.

La présente invention concerne donc les composés de formule générale I : 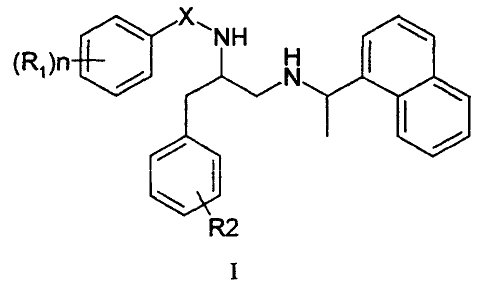 dans laquelle :
le groupe X représente un groupe SO₂ ou CH₂,
le groupe R1 représente un atome d'hydrogène ou d'halogène ou un groupe alkoxy, aryle, aralkyle ou un groupe alkyle substitué ou non par un ou plusieurs atomes d'halogènes,
n est égale à 0, 1 ou 2,
et le groupe R2 représente un atome d'hydrogène ou d'halogène ou un groupe alkyle ou alkoxy,
et leur sel avec un acide pharmaceutiquement acceptable.

De préférence, les composés selon l'invention sont représentés par la formule générale (II) : 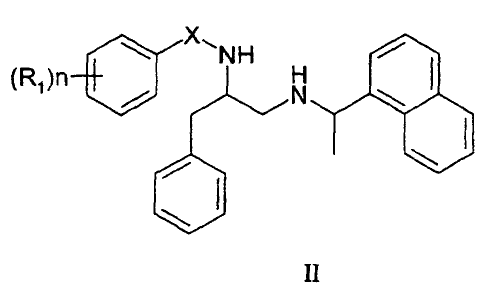 dans laquelle :
le groupe X représente un groupe SO₂ ou CH₂,
le groupe R1 représente un atome d'hydrogène ou d'halogène ou un groupe alkoxy, aryle, aralkyle ou un groupe alkyle substitué ou non par un ou plusieurs atomes d'halogènes,
et n est égale à 0, 1 ou 2.

De façon encore plus préférentiel, le groupe R1 est un noyau benzo ou alkylbenzo fusionné

Des exemples de composés préférés selon l'invention sont ceux ayant pour formule (III) : 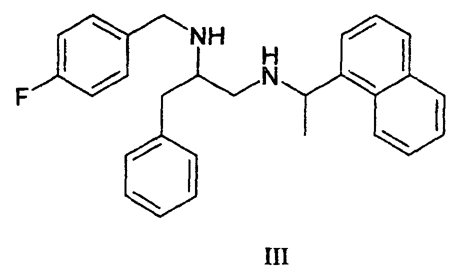 ou (IV): 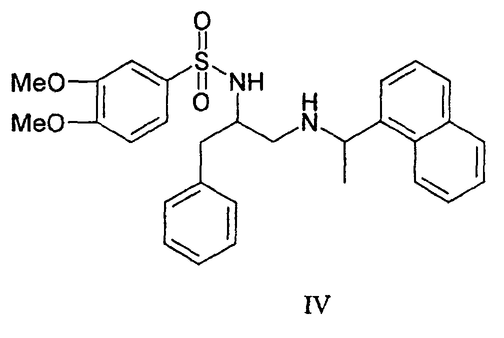 ou (V): 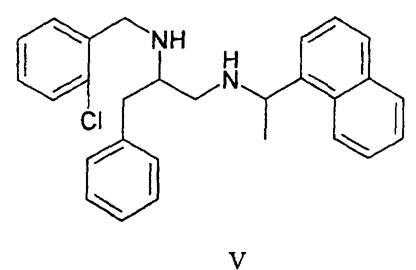 ou (VI) :

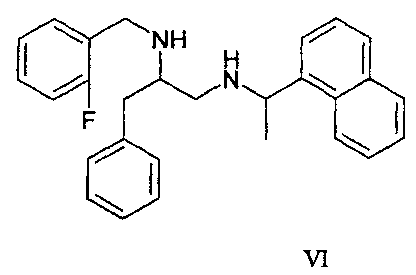

Les acides pharmaceutiquement acceptables sont des acides non toxiques, y compris les acides organiques et inorganiques. De tels acides incluent l'acide acétique, benzènesulfonique, benzoïque, citrique, éthanesulfonique, fumarique, gluconique, glutamique, bromhydrique, chlorhydrique, lactique, maléique, malique, mandélique, méthanesulfonique, mucique, nitrique, pamoique, pantothénique, phosphorique, succinique, sulfurique, tartarique et paratoluènesulfonique. L'acide chlorhydrique est particulièrement préféré.

Par le terme de groupe alkyle, on entend les groupes alkyles de 1 à 4 atomes de carbones, linéaires ou ramifiés, substitués ou non substitués. Un exemple préféré de groupes alkyles est le groupes CH₃. Un exemple préféré de groupe alkyle substitué par des atomes d'halogène est le groupe CF₃.

Par le terme de groupe alkoxy, on entend les groupes alkoxys de 1 à 4 atomes de carbones, linéaires ou ramifiés, substitués ou non substitués. Un exemple préférés de groupes alcényles est OCH₃.

Par le terme de groupes aryles on entend des cycles aromatiques ayant de 4 à 8 atomes de carbones, substitués ou non substitués, ayant un seul ou plusieurs noyau aromatiques. Les cycles aromatiques peuvent être accolés ou fusionnés entre eux ou fusionné au cycle aromatique présent dans la molécule de départ. Un exemple de groupe aryle préféré est un groupe benzo fusionné.

Par le terme de groupes aralkyles on entend des groupes aryles, définis comme ci-dessus, liés au groupe phényle par l'intermédiaire d'un groupe alkyle défini comme précédemment.

Des exemples préférés d'atome d'halogène sont Cl et F.

Les composés selon l'invention possèdent tous un centre d'asymétrie et peuvent donc exister sous forme d'isomères optiques. La présente invention comprend aussi bien ces isomères soit séparément soit en tant que mélange.

La présente invention concerne également le mode de préparation de ces composés qui peut être le suivant :
- Dans le cas où X représente le groupe SO₂, le procédé de préparation comporte les étapes suivantes :
   a) le composé de formule (VII) : 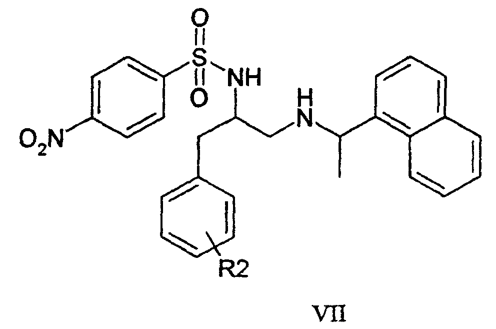 dans laquelle :
      le groupe R2 représente un atome d'hydrogène ou d'halogène ou un groupe alkyle ou alkoxy
      subit une réaction de déprotection
   b) un groupement arylsulfonyle est introduit sélectivement sur une de ses fonctions amines du composé obtenu.
- Dans le cas où X représente le groupe CH2 le procédé de préparation comporte les étapes suivantes :
   a) un groupement arylbenzyle est introduit sélectivement sur une des fonctions amines du composé de formule (VII)
   b) le composé obtenu subit une réaction de déprotection.

Le composé de formule (VII) est obtenu par ouverture nucléophile par la 1-(1-naphtyl)éthylamine de la 2-benzyl-1-(*p*-nitrobenzènesulfonyl)aziridine de formule générale (VIII) : 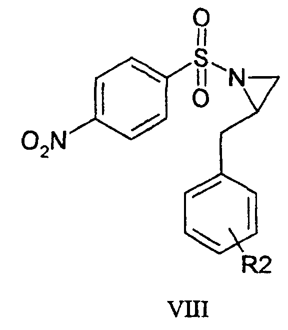 dans laquelle :
le groupe R2 représente un atome d'hydrogène ou d'halogène ou un groupe alkyle ou alkoxy.

Le composé de formule (VIII) est obtenu par réaction entre une oléfine de formule générale (IX) : 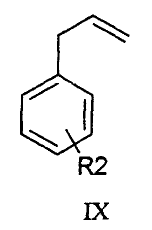 dans laquelle :
le groupe R2 représente un atome d'hydrogène ou d'halogène ou un groupe alkyle ou alkoxy
et PhI=NSO₂Ph-*p*-NO₂ en présence de Cu^{I ou II} et de CH₃CN.

La présente invention concerne également les compositions pharmaceutiques comprenant à titre de principe actif un des composés définis ci-dessus et un excipient approprié. Ces compositions peuvent être formulées pour l'administration aux mammifères, y compris l'homme. La posologie varie selon le traitement et selon l'affection en cause. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriés comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

La présente invention concerne également l'utilisation des ces composés et des compositions pharmaceutiques les comprenant comme modulateur de l'activité du CaSR
Le CaSR peut se trouver dans la glande parathyroïde, la thyroïde, les cellules osseuses, l'estomac, le poumon, le rein, l'hypophyse, le cerveau, l'hypothalamus, les aires olfactives ou l'hippocampe.

Les composés selon la présente invention sont de préférence plus sélectifs dans leur utilisation vis à vis des récepteurs de la parathyroïde comparativement à ceux de la glande thyroïde.

Les composés selon l'invention et les compositions pharmaceutiques les comprenant peuvent être utilisés comme médicament, en particulier pour le traitement des maladies ou des désordres physiologiques liés à des perturbations de l'activité des CaSR De façon encore plus particulière, ces maladies ou désordres physiologiques sont du type hyperparathyroïdies primaires ou secondaires, ostéoporose, maladies cardio-vasculaires, gastro-intestinales, endocrines, neurodégénératives ou certains cancers où les ions (Ca²⁺)ₑ sont anormalement élevés. L'hyperparathyroïdie secondaire est plus particulièrement observée lors d'insuffisance rénale chronique.

La préparation des composés selon l'invention, décrite dans le schéma ci-dessous, implique l'ouverture nucléophile par la 1-(1-naphtyl)éthylamine (5) de la 2-benzyl-1-(*p*-nitrobenzènesulfonyl)aziridine (4), synthétisée en une étape à partir de l'oléfine 3. Selon l'ordre de la séquence de réactions de déprotection et protection effectuée sur le dérivé 6, il est alors possible d'introduire sélectivement un groupement arylsulfonyle ou benzyle sur une des fonctions amines. Un des intérêts de cette chimie originale est de pouvoir incorporer facilement un grand nombre de substituants variés R1 et R2.

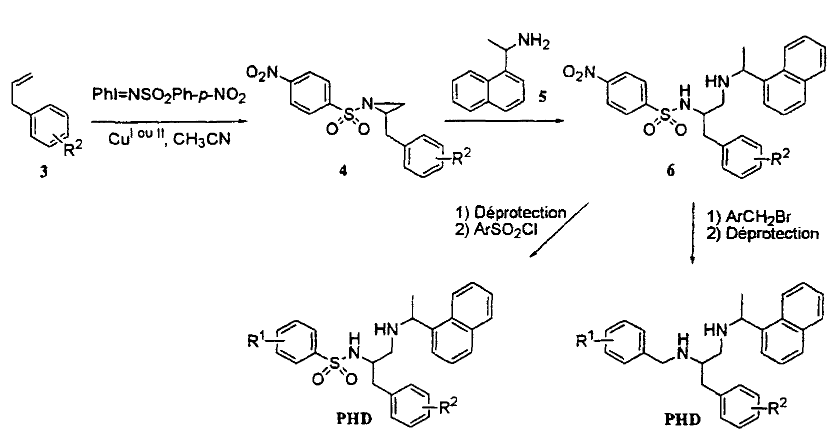

Les exemples de synthèse suivants, donnés à titre non limitatif, illustrent l'invention.

### Synthèse de la 2-Benzyl-1-(p-nitrobenzènesulfonyl)aziridine 4.

A une solution de trifluorométhanesulfonate de cuivre (I) (500 mg ; 0,9 mmole) dans 20 ml d'acétonitrile distillé, en présence de tamis moléculaire activé, sont successivement ajoutés, à 0°C sous argon, l'allylbenzène (1,60 ml ; 12 mmoles) et, par portions de 1 g sur une période de 36 heures, PhI=NSO₂Ph-*p*-NO₂ (4,85 g ; 12 mmoles). Le mélange hétérogène, de couleur marron puis verte, est agité à 0°C pendant 72 heures avant d'être filtré sur silice (Eluant : acétate d'éthyle) pour enlever le tamis moléculaire et les sels de cuivre. Après évaporation des solvants, le résidu huileux jaune est purifié par chromatographie sur silice (Eluant : heptane / acétate d'éthyle : 7 / 2) pour donner 1,76 g (5,53 mmoles ; 46%) d'un solide légèrement coloré.
*Point de fusion :* 107°C (litt : 107-108 °C).

### Synthèse du N¹[1-(1-naphtyl)éthyl]-N²-(4-nitrobenzènesulfonyl)-3-phényl-propane-1,2-diamine 6.

A une solution de l'aziridine 4 (956 mg ; 3,0 mmoles) dans 7 ml de THF sont successivement additionnées la triéthylamine (0,020 ml ; 0,15 mmole) et la 1-(1-naphtyl)éthylamine 5 (0,970 ml ; 6,00 mmoles). Après 2 jours d'agitation à température ambiante, le milieu est concentré avant d'être purifié sur colonne de silice (Eluant : heptane / acétate d'éthyle : 2 / 1). 1,38 g (2,82 mmoles ; 94%) du composé 6 est isolé sous la forme d'une mousse jaune.
*Point de fusion* : 125-126°C
*Analyse élémentaire* : C₂₇H₂₇N₃O₄S. 1/3 H₂O : Calculé : C, 65,44 ; H, 5,63 ; N, 8,48 ; S, 6,47. Trouvé : C, 65,27 ; H, 5,28 ; N, 8,36 ; S, 6,86.

### Synthèse du N²(3,4-diméthoxybenzènesulfonyl)-N¹[1-(1-naphtyl)éthyl]-3-phénylpropane-1,2-diamine PHD 321.

Le sulfonamide 6 (435 mg ; 0,888 mmole) est chauffé, à 50°C sous argon, dans une solution de thiophénol (0,270 ml ; 2,63 mmoles) et de carbonate de potassium (490 mg ; 3,54 mmoles) dans 10 ml d'un mélange 49/1 d'acétonitrile/DMSO. Après 6 heures de réaction, le milieu est concentré avant d'être purifié sur colonne de silice (Eluant : acétate d'éthyle / méthanol : 7 / 3) pour donner 250 mg (0,821 mmole ; 92%) de produit de déprotection.
94 mg (0,308 mmole) de ce composé en solution dans 3 ml de dichlorométhane sont mis en réaction en présence de 2 équivalents de triéthylamine (0,087 ml ; 0,619 mmole) et de 1,1 équivalent de chlorure de 3,4-diméthoxybenzènesulfonyle (80 mg ; 0,34 mmole). Après 24 heures d'agitation à température ambiante, le mélange est purifié sur colonne de silice (Eluant : heptane / acétate d'éthyle : 40 /60) pour donner 140 mg (0,277 mmole ; 90%) de PHD 321 sous la forme d'une huile incolore. Celle-ci, traitée par une solution d'acide chlorhydrique gazeux dans le dichlorométhane, est transformée en solide blanc correspondant au chlorhydrate du produit attendu.
*Point de fusion :* 186°C
*Spectrométrie de masse (IC)* : m/z : 505 [M+H]⁺

### Synthèse du N²-(2-Chlorobenzyl)-N¹-[1-(1-naphtyl)éthyl]-3-phénylpropane-1,2-diamine PHD 307.

Le sulfonamide 6 (88 mg ; 0,180 mmole) en solution dans 1,5 ml de DMF est traité à 0°C sous argon par 2 équivalents de K₂CO₃ (50 mg ; 0,361 mmole) et 1,1 équivalent de bromure de 2-chlorobenzyle (0,026 ml ; 0,200 mmole). Après 6 heures de réaction de 0°C à température ambiante, le milieu est filtré sur colonne de silice (Eluant : heptane / acétate d'éthyle : 6 / 1) pour conduire à 81 mg (0,132 mmole ; 73%) d'un solide blanc. Ce dernier est chauffé à 50°C sous argon dans un mélange de thiophénol (3 eq.) et de K₂CO₃ (4 eq.) en solution dans 1,5 ml d'acétonitrile/DMSO : 49/1. Après 20 heures de réaction, la chromatographie sur colonne de silice (Eluant : heptane / acétate d'éthyle : 1 / 3) permet d'isoler 45 mg (0,105 mmole ; 80%) d'une huile incolore. Par traitement dans une solution d'HCl_{gaz} dans CH₂Cl₂, le chlorhydrate de PHD 307 est isolé sous la forme d'une poudre blanche.
*Point de fusion* : 129°C
*Spectrométrie de masse (FAB)* : m/z : 429 [M+H]⁺

### Activité sur des cellules transfectées exprimant le récepteur sensible aux ions (Ca²⁺)e

L'activité calcimimétique des composés a été estimée en mesurant l'accumulation d'inositols phosphates tritiés induite par 10 µM de chacun des composés en présence de 2 mM de Ca²⁺ dans les cellules CHO(CaSR) (Ferry et al, Biochem Biophys Res Commun, 1997).

Cette activation a été comparée à celle induite par le composé NPS-R- 568, un calcimimétique de référence et utilisé à une concentration de 10 µM (Tableau 1) (Ferry et al, Biochem Biophys Res Commun, 1997 ; Nemeth et al, Proc Natl Acad Sci USA, 1997).

Les composés PHD 307, 320, 321 et 323, utilisés à une concentration de 10 µM présentent une activité allant de 90 à 100 % de celle obtenue par 10 mM de Ca²⁺, alors que le NPS-R-568 présente une activité de 100 % à la même concentration. Dans cette série chimique certains composés tels PHD 90, 128, 129, 125, sont dépourvus d'activité calcimimétique à cette concentration (Tableau 1).

La comparaison de la structure des composés PHD 181 et PHD 182 d'une part et PHD 206 et PHD 217 d'autre part, indique que le groupement naphtyle conduit à une activité supérieure par rapport au groupement 3-méthoxyphényle.

Dans le tableau 1, l'activité calcimimétique des composés PHD est comparée à celle d'un composé de référence, le NPS-R-568 utilisé à la même concentration et dans les mêmes conditions expérimentales. Cette activité est exprimée en pourcentage de l'activité de 10 mM de Ca²⁺. Les moyennes ± erreurs standards de 2 à 5 manipulations indépendantes sont indiquées. Les expériences ont été réalisées en présence de 2 mM de Ca²⁺.

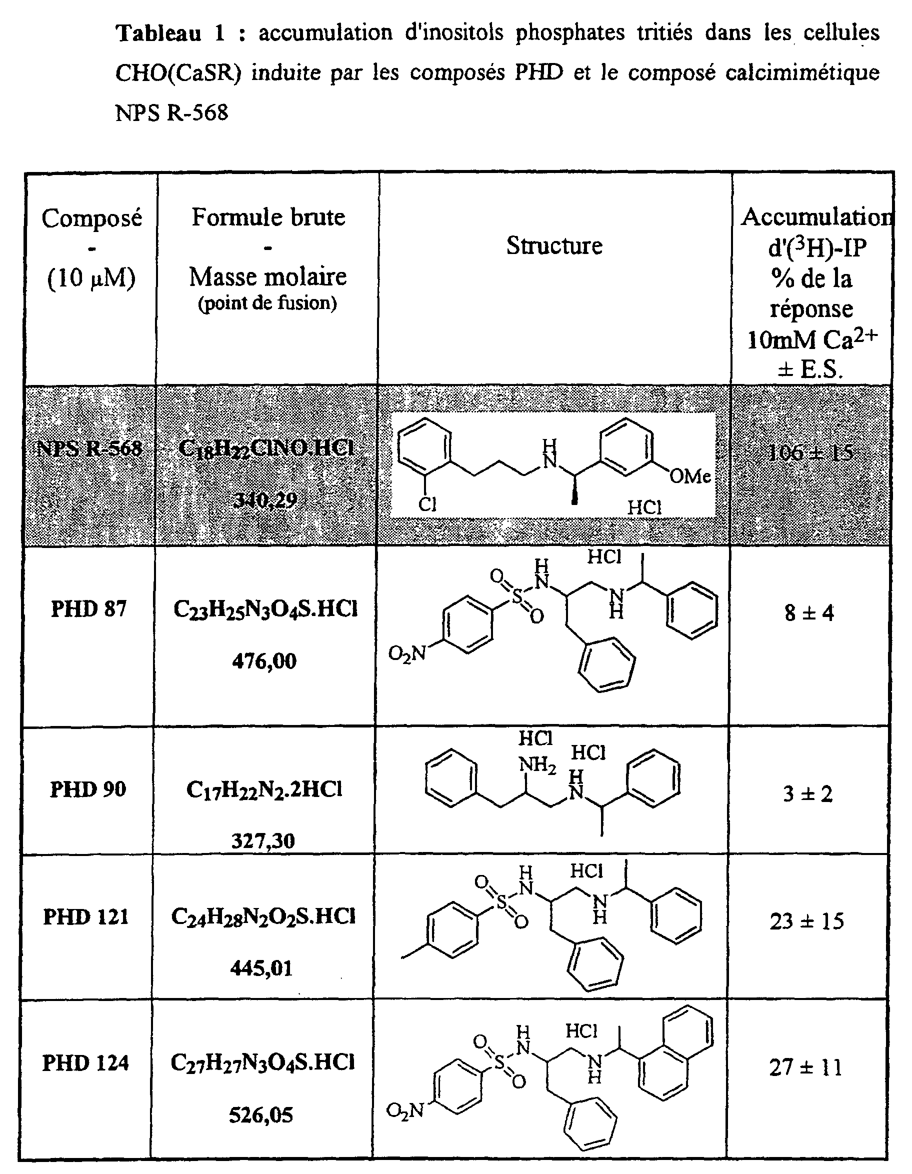

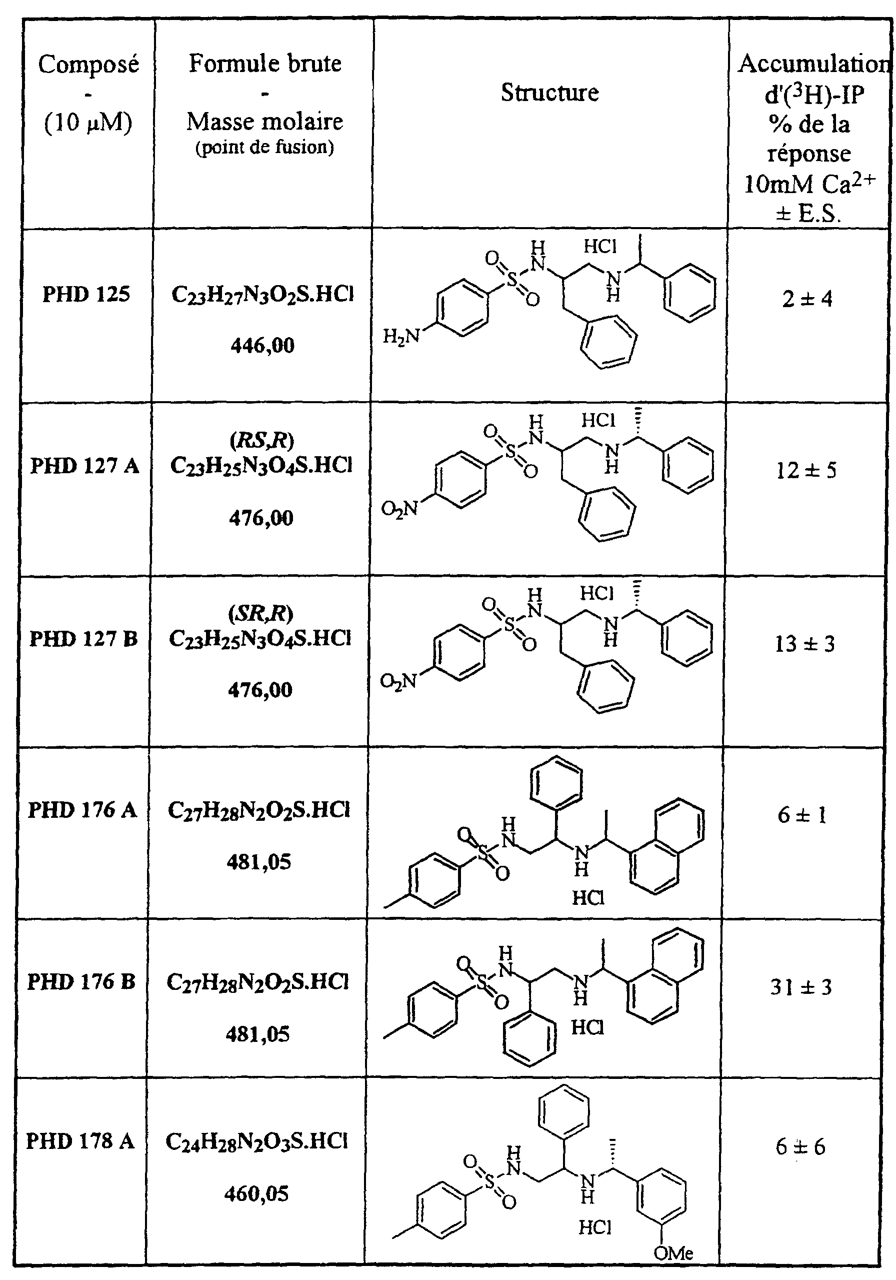

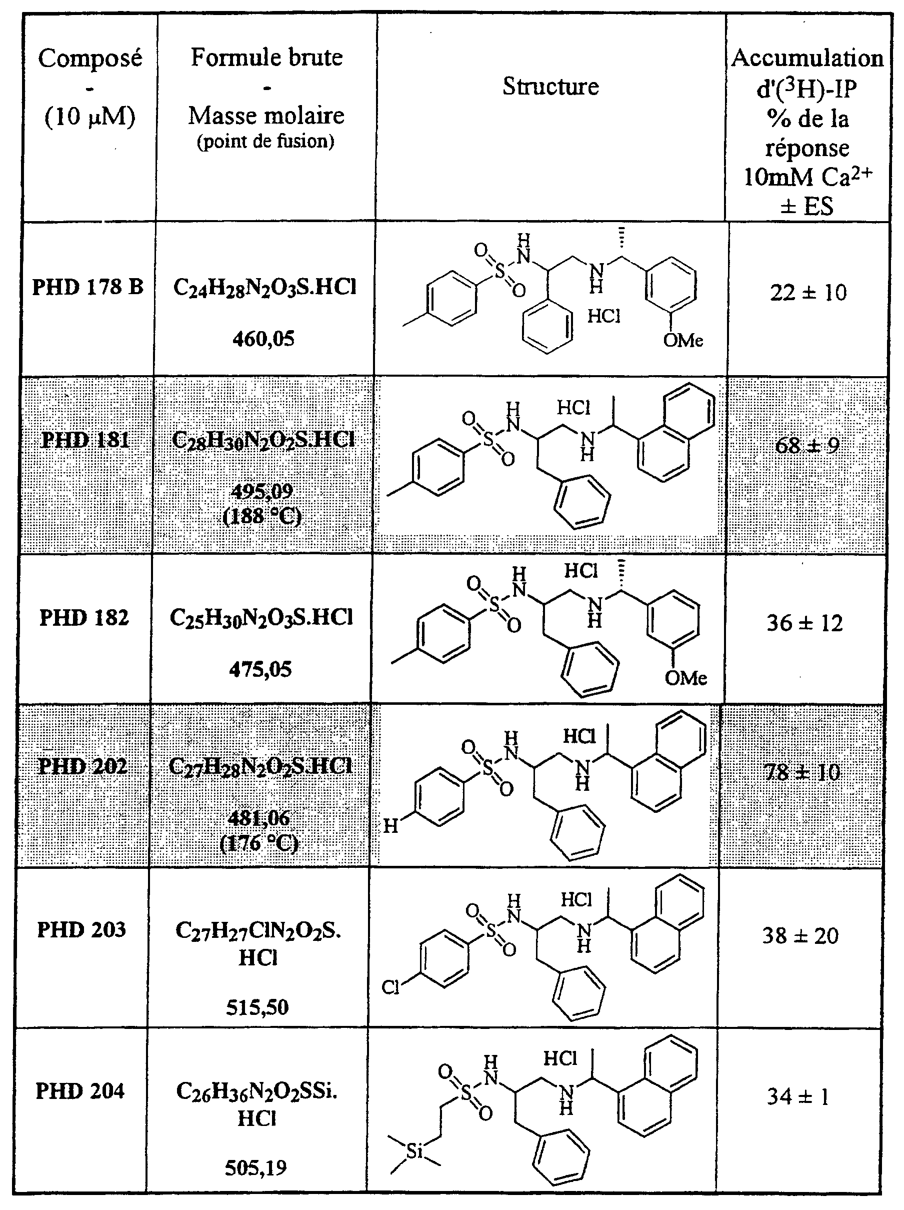

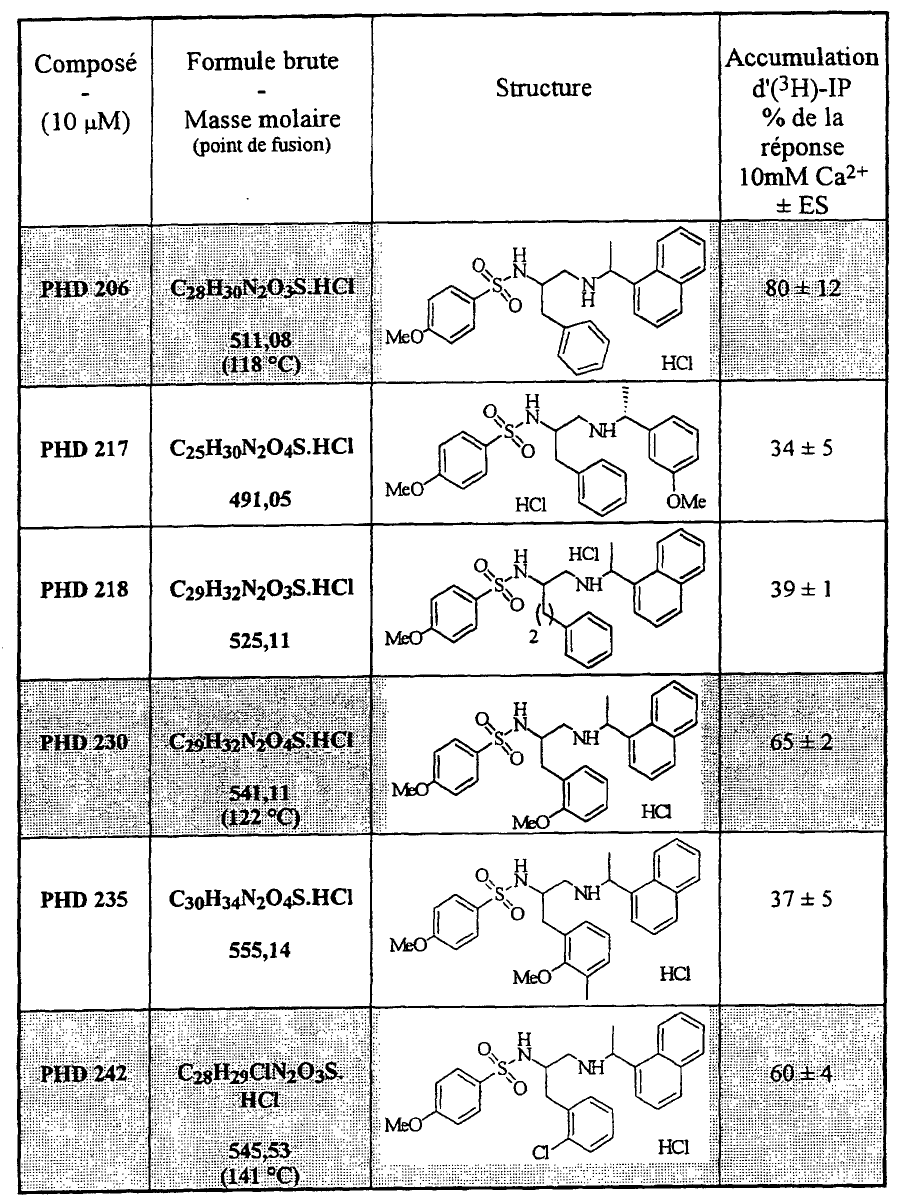

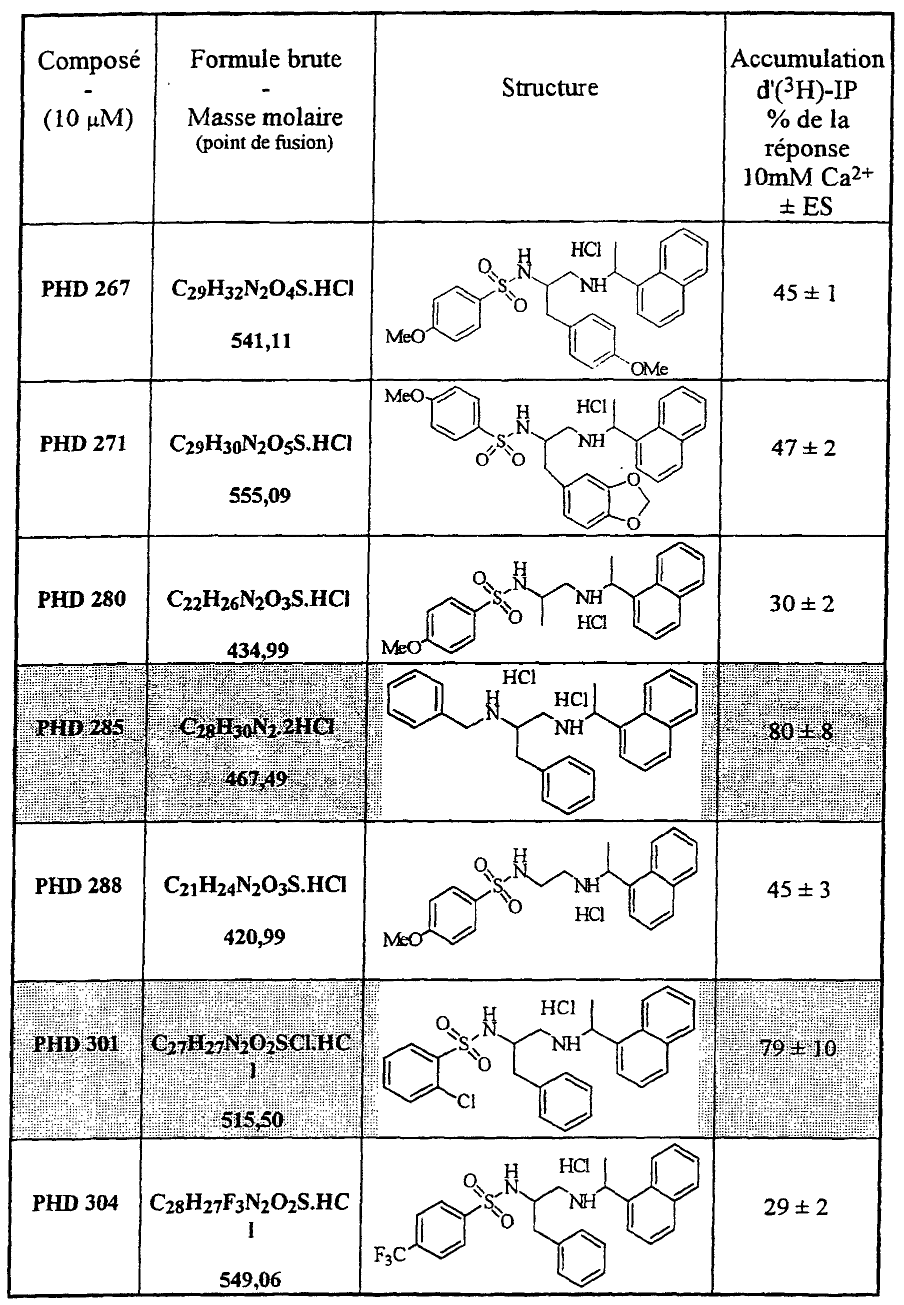

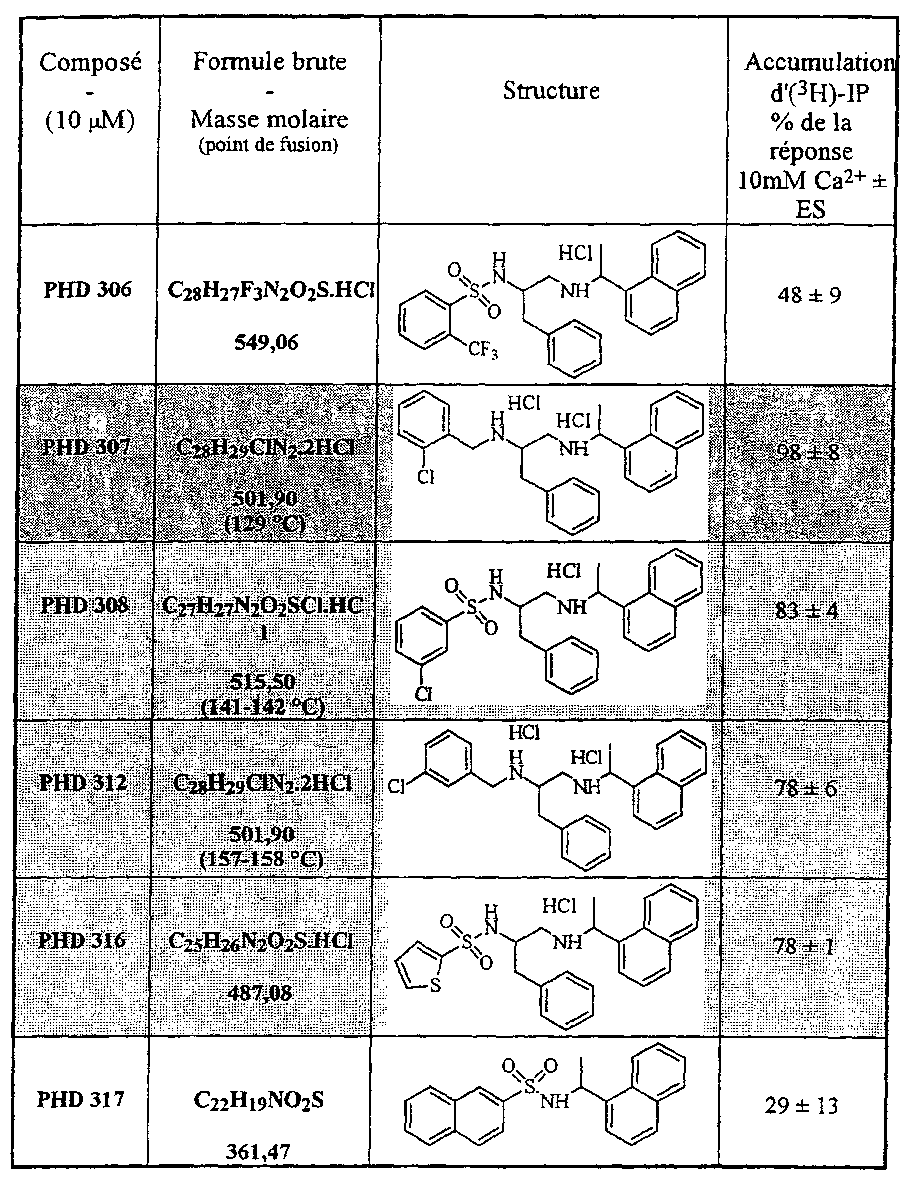

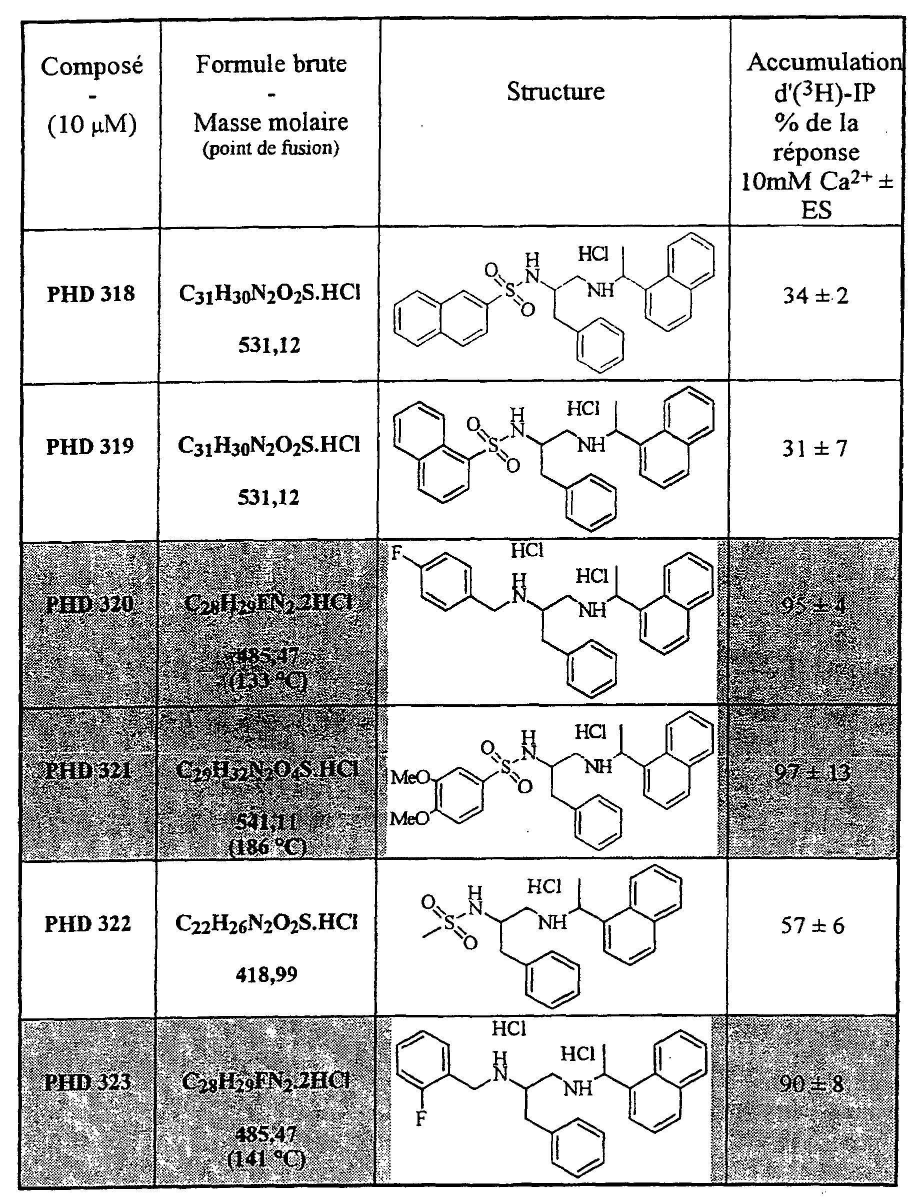

Ces exemples de composés et les résultats obtenus avec eux sont indiqués à titre non limitatif et illustrent l'invention.

### Spécificité de l'activité de ces molécules

Les molécules PHD, utilisées à une concentration de 10 µM ne conduisent pas ou peu à l'accumulation d'[³H]IP dans des cellules CHO(WT*) témoins ce qui suggère leur spécificité d'action vis-à-vis du CaSR (Tableau 2). Les cellules CHO(WT*) ont été tranfectées avec le plasmide seul et n'expriment pas le CaSR.

L'accumulation d'inositols phosphates tritiés est exprimée en pourcentage du taux de base observé en présence de 2 mM Ca²⁺ (100 %) dans les cellules CHO(WT*) ou CHO(CaSR). Les composés PHD 301, 307, 308, 312 et 321 et NPS-R-568 induisent peu ou pas d'accumulation d'[³H]IP dans les cellules CHO(WT*). Les composés PHD 301, 307, 308, 312, et 321 et NPS-R-568 induisent une forte accumulation d'[³H]IP dans les cellules CHO(CaSR).

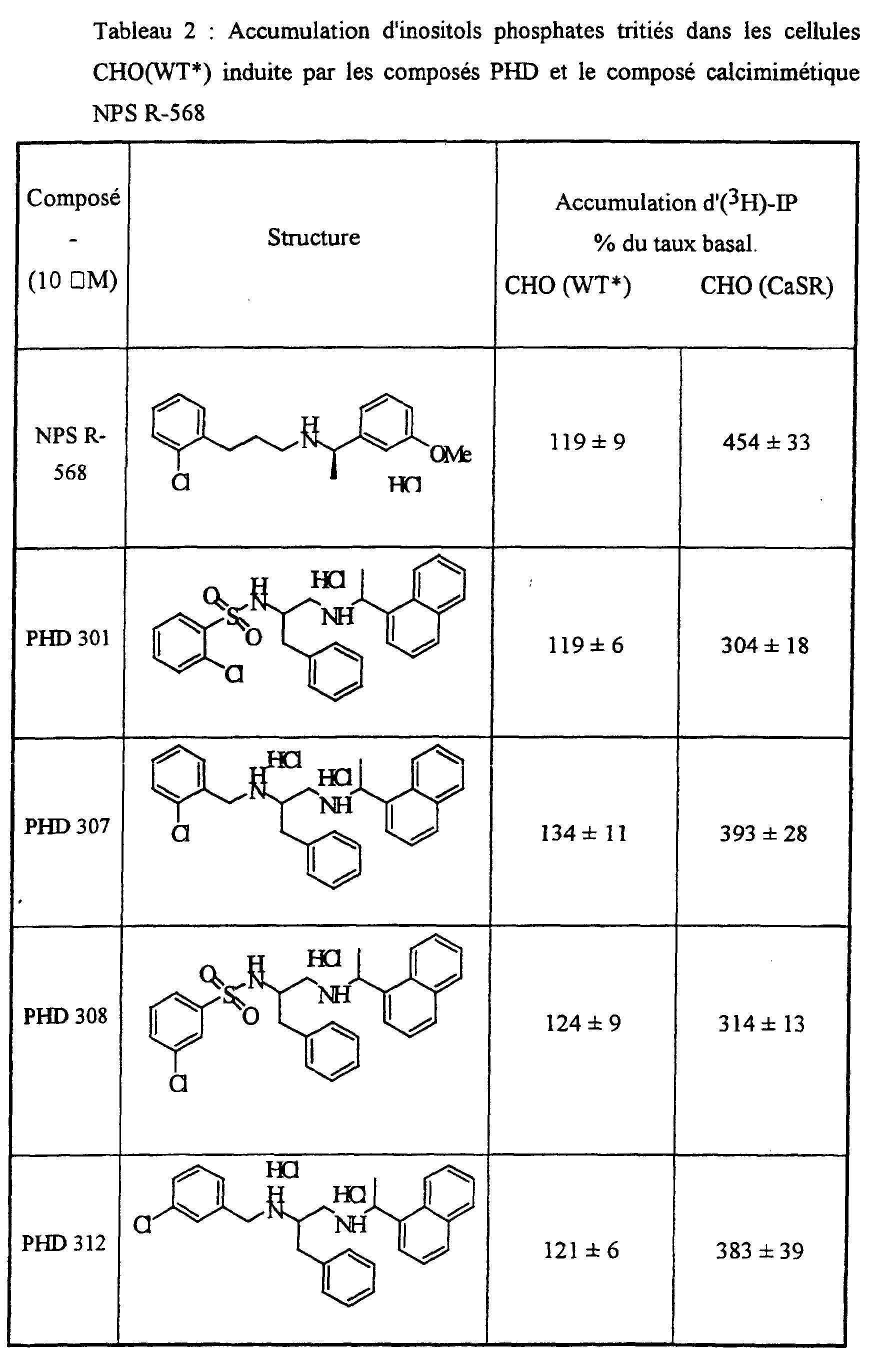

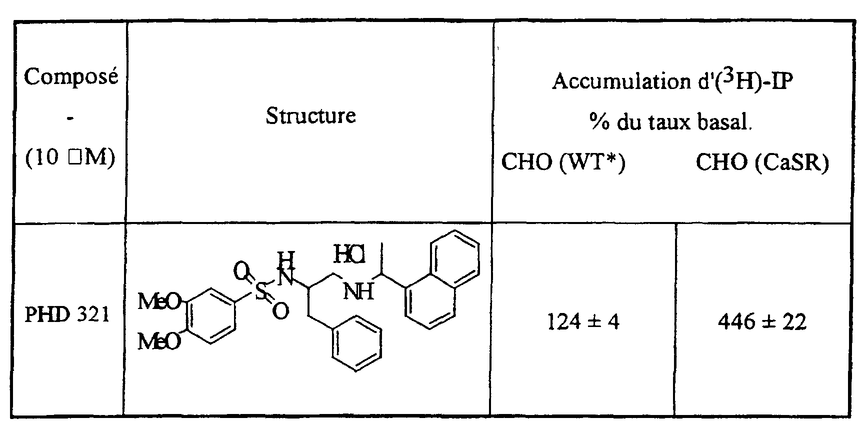

Ces exemples de composés et les résultats obtenus avec eux sont indiqués à titre non limitatif et illustrent l'invention.

## Revendications

1. Arylalkyl-1,2-diamine de formule générale (I) : 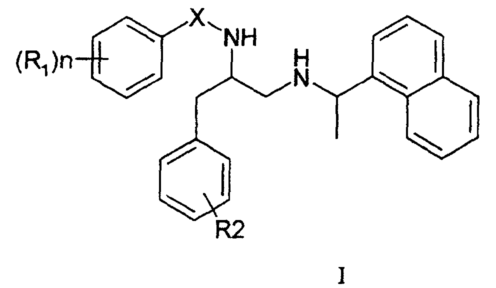 dans laquelle :
le groupe X représente un groupe SO₂ ou CH₂,
le groupe R1 représente un atome d'hydrogène ou d'halogène ou un groupe alkoxy, aryle, aralkyle ou un groupe alkyle substitué ou non par un ou plusieurs atomes d'halogènes,
n est égale à 0, 1 ou 2,
et le groupe R2 représente un atome d'hydrogène ou d'halogène ou un groupe alkyle ou alkoxy,
et leurs sel avec un acide pharmaceutiquement acceptable, sous forme de mélange racémique ou de leurs isomères optiquement purs.

2. Arylalkyl-1,2-diamine selon la revendication 1 **caractérisé en ce qu'**il est représenté par la formule générale (II) : 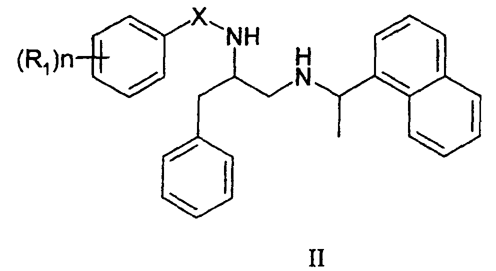 dans laquelle :
le groupe X représente un groupe SO₂ ou CH₂,
le groupe R1 représente un atome d'hydrogène ou d'halogène ou un groupe alkoxy, aryle, aralkyle ou un groupe alkyle substitué ou non par un ou plusieurs atomes d'halogènes,
et n est égale à 0, 1 ou 2.

3. Arylalkyl-1,2-diamine selon les revendications 1 et 2 **caractérisé en ce que** le groupe R1 est un noyau benzo ou alkylbenzo fusionné

4. Arylalkyl-1,2-diamine selon les revendications 1 et 2 **caractérisé en ce qu'**il est représenté par la formule (III) :

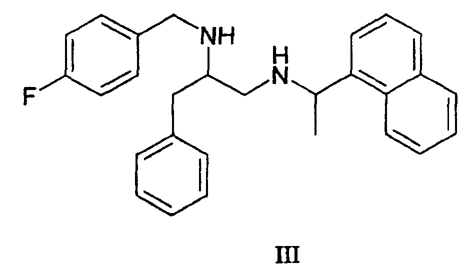

5. Arylalkyl-1,2-diamine selon les revendications 1 et 2 **caractérisé en ce qu'**il est représenté par la formule (IV):

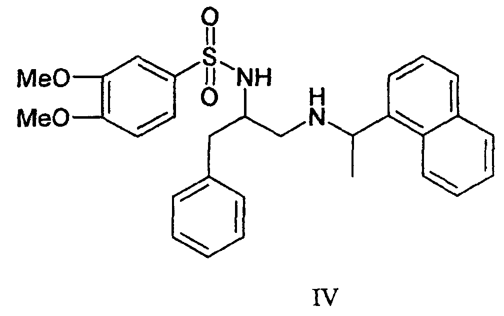

6. Arylalkyl-1,2-diamine selon les revendications 1 et 2 **caractérisé en ce qu'**il est représenté par la formule (V) :

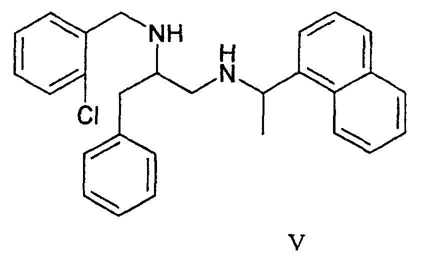

7. Arylalkyl-1,2-diamine selon les revendications 1 et 2 **caractérisé en ce qu'**il est représenté par la formule (VI) :

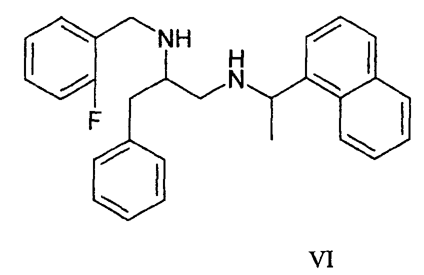

8. Procédé de préparation des arylalkyl-1, 2-diamines de formule (I) dans laquelle X représente le groupe SO₂ selon les revendications 1 à 3 et 5 **caractérisé en ce qu'**il comporte les étapes suivantes :
a) le composé de formule (VII) : 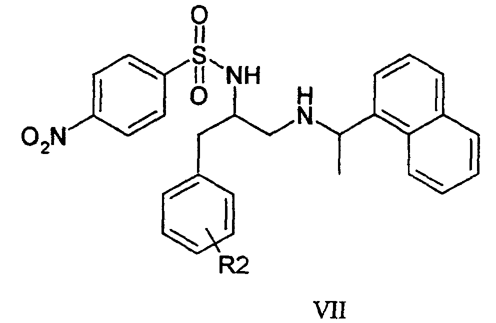 dans laquelle :
le groupe R2 représente un atome d'hydrogène ou d'halogène ou un groupe alkyle ou alkoxy
subit une réaction de déprotection
b) un groupement arylsulfonyle est introduit sélectivement sur une de ses fonctions amines du composé obtenu.

9. Procédé de préparation des arylalkyl-1,2-diamines de formule (I) dans laquelle X représente le groupe CH2 selon les revendications 1 à 4, 6 et 7 **caractérisé en ce qu'**il comporte les étapes suivantes :
a) un groupement arylbenzyle est introduit sélectivement sur une des fonctions amines du composé de formule (VII)
b) le composé obtenu subit une réaction de déprotection.

10. Procédé de préparation selon les revendications 8 et 9 **caractérisé en ce que** le composé de formule (VII) est obtenu par ouverture nucléophile par la 1-(1-naphtyl)éthylamine de la 2-benzyl-1-(*p*-nitrobenzènesulfonyl)aziridine de formule générale (VIII) : 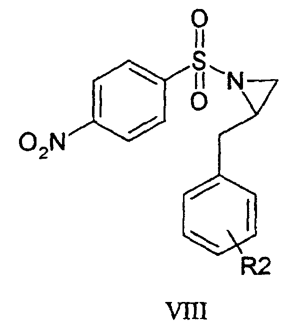 dans laquelle :
le groupe R2 représente un atome d'hydrogène ou d'halogène ou un groupe alkyle ou alkoxy.

11. Procédé de préparation selon les revendications 8 à 10 **caractérisé en ce que** le composé de formule (VIII) est obtenu par réaction entre une oléfine de formule générale (IX) : 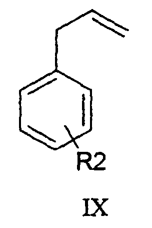 dans laquelle :
le groupe R2 représente un atome d'hydrogène ou d'halogène ou un groupe alkyle ou alkoxy
et PhI=NSO₂Ph-*p*-NO₂ en présence de Cu^{I ou II} et de CH₃CN.

12. Composition pharmaceutique comprenant un composé selon les revendications 1 à 7 et un support pharmaceutique approprié.

13. Composé selon les revendications 1 à 7 et 12 pour son utilisation comme médicament.

14. Utilisation des composés selon les revendications 1 à 7 et 12 pour la fabrication d'un médicament destiné au traitement des maladies ou des désordres physiologiques liés à des perturbations de l'activité des CaSR.

15. Utilisation selon la revendication 14 **caractérisée en ce que** les maladies ou les désordres physiologiques sont du type hyperparathyroïdies primaires ou secondaires, ostéoporose, maladies cardio-vasculaires, gastro-intestinales, endocrines, neurodégénératives ou certains cancers où les ions (Ca²⁺)ₑ sont anormalement élevés.

16. Utilisation selon la revendication 15 **caractérisée en ce que** l'hyperparathyroïdie secondaire est observée lors d'insuffisance rénale chronique.

## Patentansprüche

1. Arylalkyl-1,2-diamin der allgemeinen Formel (I): 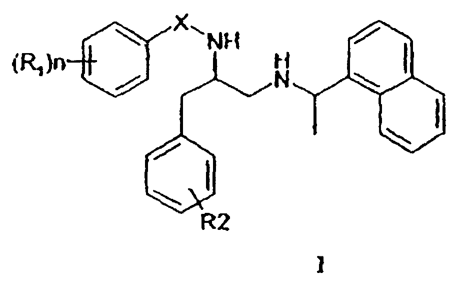 in welcher:
die Gruppe X für eine Gruppe SO₂ oder CH₂ steht,
die Gruppe R1 für ein Wasserstoff- oder Halogenatom oder eine Alkoxy-, Aryl-, Arylalkylgruppe oder eine Alkylgruppe, die durch ein oder mehrere Halogenatome substituiert oder nicht substituiert ist, steht,
n gleich 0, 1 oder 2 ist
und die Gruppe R2 für ein Wasserstoff- oder Halogenatom oder eine Alkyl- oder Alkoxygruppe steht,
und deren Salz mit einer pharmazeutisch annehmbaren Säure, in Form einer racemischen Mischung oder von deren optisch reinen Isomeren.

2. Arylalkyl-1,2-diamin nach Anspruch 1, **dadurch gekennzeichnet, dass** es angegeben wird durch die allgemeine Formel (II): 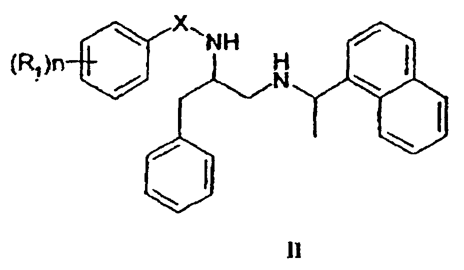 in welcher:
die Gruppe X für eine Gruppe SO₂ oder CH₂ steht,
die Gruppe R1 für ein Wasserstoff- oder Halogenatom oder eine Alkoxy-, Aryl-, Arylalkylgruppe oder eine Alkylgruppe, die durch ein oder mehrere Halogenatome substituiert oder nicht substituiert ist, steht und
n gleich 0, 1 oder 2 ist.

3. Arylalkyl-1,2-diamin nach den Ansprüchen 1 und 2, **dadurch gekennzeichnet, dass** die Gruppe R1 ein kondensierter Benzooder Alkylbenzoring ist.

4. Arylalkyl-1,2-diamin nach den Ansprüchen 1 und 2, **dadurch gekennzeichnet, dass** es angegeben wird durch die Formel (III):

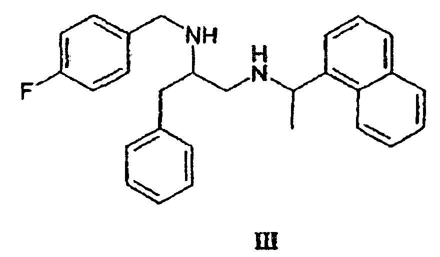

5. Arylalkyl-1,2-diamin nach den Ansprüchen 1 und 2, **dadurch gekennzeichnet, dass** es angegeben wird durch die Formel (IV):

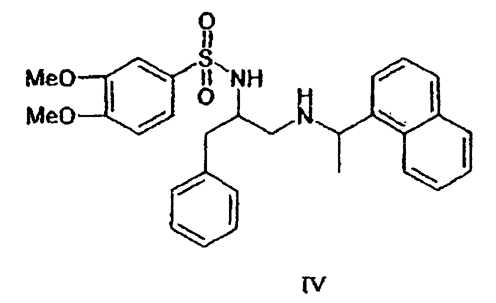

6. Arylalkyl-1,2-diamin nach den Ansprüchen 1 und 2, **dadurch gekennzeichnet, dass** es angegeben wird durch die Formel (V):

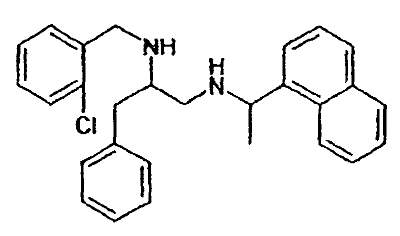

7. Arylalkyl-1,2-diamin nach den Ansprüchen 1 und 2, **dadurch gekennzeichnet, dass** es angegeben wird durch die Formel (VI):

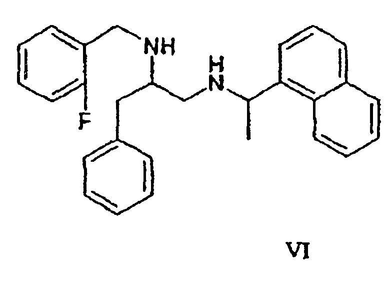

8. Verfahren zur Herstellung der Arylalkyl-1,2-diamine der Formel (I), in welcher X für die Gruppe SO₂ steht, nach den Ansprüchen 1 bis 3 und 5, **dadurch gekennzeichnet, dass** es die folgenden Schritte umfasst:
a) die Verbindung der Formel (VII): 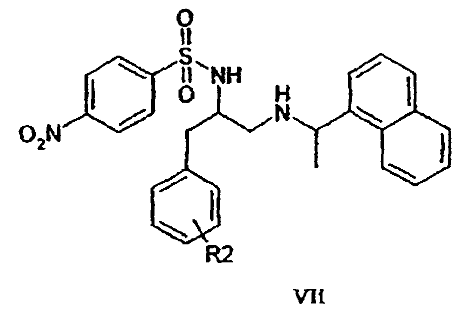 in welcher:
die Gruppe R2 für ein Wasserstoff- oder Halogenatom oder eine Alkyl- oder Alkoxygruppe steht,
durchläuft eine Schutzgruppenentfernungsreaktion,
b) eine Arylsulfonylgruppe wird selektiv an einer von deren Aminfunktionen der erhaltenen Verbindung eingeführt.

9. Verfahren zur Herstellung der Arylalkyl-1,2-diamine der Formel (I), in welcher X für die Gruppe CH₂ steht, nach den Ansprüchen 1 bis 4, 6 und 7, **dadurch gekennzeichnet, dass** es die folgenden Schritte umfasst:
a) eine Arylbenzylgruppe wird selektiv an einer der Aminfunktionen der Verbindung der Formel (VII) eingeführt,
b) die erhaltene Verbindung durchläuft eine Schutzgruppenentfernungsreaktion.

10. Herstellungsverfahren nach den Ansprüchen 8 und 9, **dadurch gekennzeichnet, dass** die Verbindung der Formel (VII) erhalten wird durch nukleophile Öffnung durch 1-(1-Naphthyl)ethylamin des 2-Benzyl-1-(*p*-nitrobenzolsulfonyl)aziridins der allgemeinen Formel (VIII): 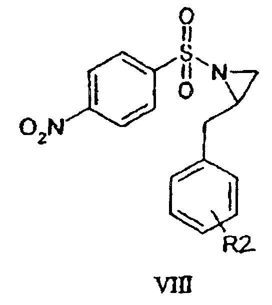 in welcher:
die Gruppe R2 für ein Wasserstoff- oder Halogenatom oder eine Alkyl- oder Alkoxygruppe steht.

11. Herstellungsverfahren nach den Ansprüchen 8 bis 10, **dadurch gekennzeichnet, dass** die Verbindung der Formel (VIII) erhalten wird durch Reaktion zwischen einem Olefin der allgemeinen Formel (IX) : 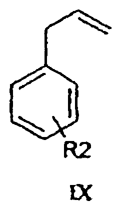 in welcher:
die Gruppe R2 für ein Wasserstoff- oder Halogenatom oder eine Alkyl- oder Alkoxygruppe steht,
und PhI=NSO₂Ph-*p*-NO₂ in Gegenwart von Cu^{I oder II} und von CH₃CN.

12. Pharmazeutische Zusammensetzung, welche eine Verbindung nach den Ansprüchen 1 bis 7 und einen geeigneten pharmazeutischen Träger umfasst.

13. Verbindung nach den Ansprüchen 1 bis 7 und 12 für eine Verwendung von dieser als Arzneimittel.

14. Verwendung der Verbindungen nach den Ansprüchen 1 bis 7 und 12 zur Herstellung eines Arzneimittels, welches für die Behandlung der Krankheiten oder physiologischen Störungen, die mit Störungen der CaSR-Aktivität verbunden sind, bestimmt ist.

15. Verwendung nach Anspruch 14, **dadurch gekennzeichnet, dass** die Krankheiten oder physiologischen Störungen vom Typ primäre oder sekundäre Hyperparathyreoidismen, Osteoporose, Herz-Kreislauf-Erkrankungen, Magen-Darm-Erkrankungen, endokrine Erkrankungen, neurodegenerative Erkrankungen oder bestimmter Krebsarten, wo die Ca(²⁺)ₑ-Ionen abnormal erhöht sind, sind.

16. Verwendung nach Anspruch 15, **dadurch gekennzeichnet, dass** der sekundäre Hyperparathyreoidismus während einer chronischen Niereninsuffizienz beobachtet wird.

## Claims

1. Arylalkyl-1,2-diamine of general formula (I): 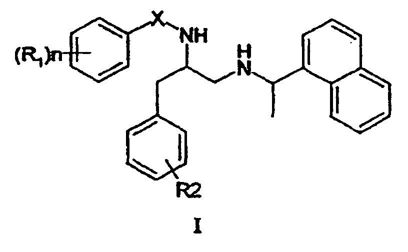 in which:
the group X represents an SO₂ or CH₂ group,
the group R1 represents a hydrogen or halogen atom or an alkoxy, aryl or aralkyl group or an alkyl group which is substituted or otherwise with one or more halogen atoms,
n is equal to 0, 1 or 2,
and the group R2 represents a hydrogen or halogen atom or an alkyl or alkoxy group,
and their salts with a pharmaceutically acceptable acid, in the form of a racemic mixture or of their optically pure isomers.

2. Arylalkyl-1,2-diamine according to Claim 1, **characterized in that** it is represented by the general formula (II): 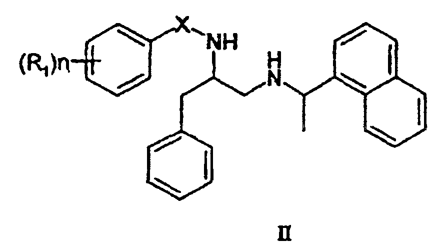 in which:
the group X represents an SO₂ or CH₂ group,
the group R1 represents a hydrogen or halogen atom or an alkoxy, aryl or aralkyl group or an alkyl group which is substituted or otherwise with one or more halogen atoms,
and n is equal to 0, 1 or 2.

3. Arylalkyl-1,2-diamine according to Claims 1 and 2, **characterized in that** the group R1 is a fused benzo or alkylbenzo nucleus.

4. Arylalkyl-1,2-diamine according to Claims 1 and 2, **characterized in that** it is represented by the formula (III):

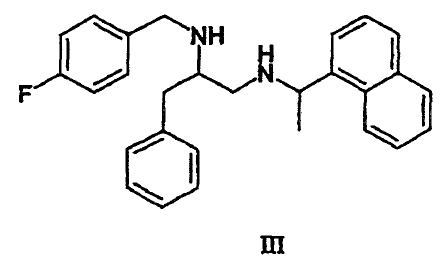

5. Arylalkyl-1,2-diamine according to Claims 1 and 2, **characterized in that** it is represented by the formula (IV):

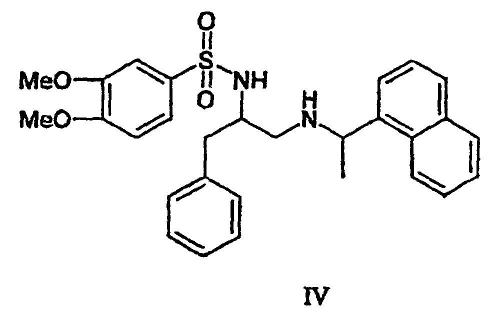

6. Arylalkyl-2,2-diamine according to Claims 1 and 2, **characterized in that** it is represented by the formula (V) :

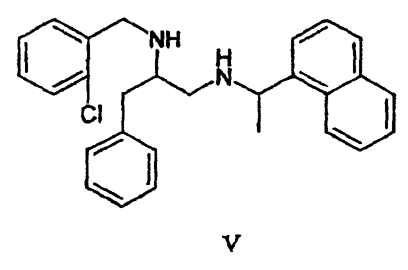

7. Arylalkyl-2,2-diamine according to Claims 1 and 2, **characterized in that** it is represented by the formula (VI):

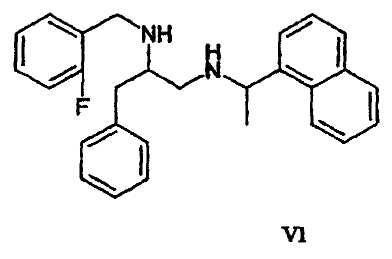

8. Method for preparing arylalkyl-1,2-diamines of formula (I) in which X represents the SO₂ group according to Claims 1 to 3 and 5, **characterized in that** it comprises the following steps:
a) the compound of formula (VII): 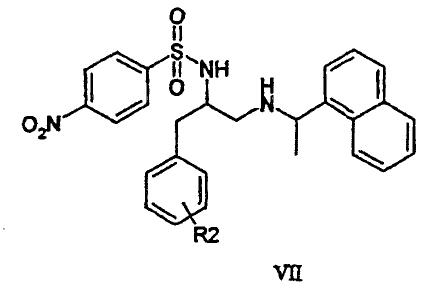 in which:
the group R2 represents a hydrogen or halogen atom or an alkyl or alkoxy group
undergoes a deprotection reaction
b) an arylsulfonyl group is selectively introduced onto one of its amine functional groups of the compound obtained.

9. Method for preparing arylalkyl-1,2-diamines of formula (I) in which X represents the CH2 group according to Claims 1 to 4, 6 and 7, **characterized in that** it comprises the following steps:
a) an arylbenzyl group is selectively introduced onto one of the amine functional groups of the compound of formula (VII)
b) the compound obtained undergoes a deprotection reaction.

10. Method of preparation according to Claims 8 and 9, **characterized in that** the compound of formula (VII) is obtained by nucleophilic opening, with 1-(1-naphthyl)ethylamine, of 2-benzyl-1-(*p*-nitro-benzenesulfonyl)aziridine of general formula (VIII): 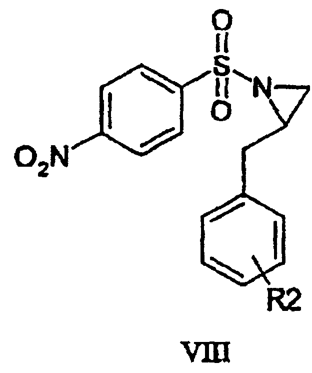 in which:
the group R2 represents a hydrogen or halogen atom or an alkyl or alkoxy group.

11. Method of preparation according to Claims 8 to 10, **characterized in that** the compound of formula (VIII) is obtained by reaction between an olefin of general formula (IX): 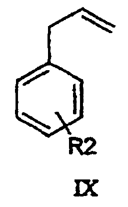 in which:
the group R2 represents a hydrogen or halogen atom or an alkyl or alkoxy group
and PhI=NSO₂Ph-*p*-NO₂ in the presence of Cu^{I or II} and of CH₃CN.

12. Pharmaceutical composition comprising a compound according to Claims 1 to 7 and an appropriate pharmaceutical carrier.

13. Compound according to Claims 1 to 7 and 12, for its use as a medicament.

14. Use of the compounds according to Claims 1 to 7 and 12, for the manufacture of a medicament intended for the treatment of physiological diseases or disorders linked to disturbances in the CaSR activity.

15. Use according to Claim 14, **characterized in that** the physiological diseases or disorders are of the type including primary or secondary hyperparathyroidism, osteoporosis or cardiovascular, gastrointestinal, endocrine or neurodegenerative diseases or certain cancers where the (Ca²⁺)ₑ ions are abnormally high.

16. Use according to Claim 15, **characterized in that** the secondary hyperparathyroidism is observed during chronic renal insufficiency.
